# EUROPEAN PATENT APPLICATION

(11) **EP 4 183 246 A1**
(43) Date of publication of application: **24.05.2023**
(21) Application number: 21841982.8
(22) Date of filing: 16.07.2021
(51) Int. Cl.: A01H 1/02, A01H 6/82

(54) **GENUS NICOTIANA F1 HYBRID AND USE THEREOF**

(30) Priority: 17.07.2020 JP 2020122683
(71) Applicant: Japan Tobacco Inc., Tokyo 105-6927 (JP)
(72) Inventor: YAMAUCHI, Shun, Tokyo 130-8603 (JP)
(74) Representative: Hoffmann Eitle
(86) International application number: PCT/JP2021/026787
(87) International publication number: WO 2022/014699

(57) **Abstract**

An object of the present invention is to provide an F1 hybrid or a portion thereof, and use of the F1 hybrid or a portion thereof. The F1 hybrid of the present invention is an F1 hybrid between Nicotiana umbratica and Nicotiana tomentosa.

## Description

### TECHNICAL FIELD

The present invention relates to a genus Nicotiana F1 hybrid and use thereof.

### BACKGROUND ART

### 1. Plants of genus Nicotiana

Plants of the genus Nicotiana (also called "Nicotiana") are widespread in the South/North American Continents, the Australian continent, and in the African continent, and currently 70 or more species are reported. Two species, Nicotiana tabacum and Nicotiana rustica, which are each used as a raw material for a tobacco product, are the best known plants of the genus Nicotiana.

With respect to wild species of the genus Nicotiana other than the two species, there are a large number of reports on genetic analysis and analysis of disease resistance including those using, as a material, a plant obtained by hybridization between wild species. Examples thereof include: Takenaka, Jap. Jour. Genet. 31. 155-161, 1956; and MULKH RAJ AHUJA, Genetica 47, 865-880, 1962. There are reports on utilizing a wild species itself as a raw material for a tobacco product, but the number of such reports is small. Examples thereof include US Patent No. 7,798,153. However, a report on using an F1 hybrid plant which is obtained by hybridizing wild species for a tobacco product has not been found so far.

### 2. Nicotiana umbratica

"Nicotiana umbratica" is a species of the genus Nicotiana, spreading in northwestern Western Australia. Nicotiana umbratica is an annual grass that has a grass height of about 50 to about 60 cm and grows thick while a large number of thin stems branch, and glandular hairs grow on the whole body to make Nicotiana umbratica sticky, and a strong scent floats. The leaf blade of Nicotiana umbratica has a wide egg shape having a length of about 8 cm, and Nicotiana umbratica has a lot of racemose flowers at the tips of branches with leaves and at the tips of branches at axillary growth. The main alkaloid is nicotine. Nicotiana umbratica is resistant to powdery mildew and downy mildew (common) (The genus Nicotiana illustrated, Japan Tobacco Inc. (edited), 180-183, 1994 March, SEIBUNDO SHINKOSHA Publishing Co., LTD.). Nicotiana umbratica has such a characteristically strong scent and is therefore considered to be preferable as a raw material for a tobacco product. However, a report on utilizing Nicotiana umbratica as a raw material for a tobacco product has not been found so far.

On the other hand, genetic analysis and the like using Nicotiana umbratica as an experimental material are reported in a large number of literatures. For example, U. Subhashini et al. have reported that hybridization between Nicotiana umbratica and another wild species of the genus Nicotiana was performed (U. Subushini, Euphytica, 23, 289-293, 1974; U. Subushini at al., Cytologia 39, 403-409, 1974; U. Subushini at al., Cytologia 40, 409-413, 1975).

### 3. Nicotiana kawakamii

"Nicotiana kawakamii" is a species of the genus Nicotiana, discovered by Kawakami Yoshiyasu et al. in Bolivia in 1968. In the place of origin, Nicotiana kawakamii is a semi-arbor shrub with a height of about 4 m, the stem is sticky, and the tip part is reddish purple. The leaf has a wide elliptic shape having a length of about 60 cm. The branch having a panicle is thick and spread, has buds and flowers spirally at the tip thereof, and has deep reddish purple capsules in line. The way the leaves grow is dense. The number of flowers is small, and the upper part of the plant body has the flowers together. The main alkaloid is nornicotine. Nicotiana kawakamii is resistant to powdery mildew and PVY (The genus Nicotiana illustrated, Japan Tobacco Inc. (edited), 48-51, 1994 March, SEIBUNDO SHINKOSHA Publishing Co., LTD.). Utilization for a tobacco product is reported in US Patent No. 7,798,153 granted to US Smokeless Tobacco Co.

### 4. Nicotiana tomentosa

"Nicotiana tomentosa" is a species of the genus Nicotiana, spreading in central to southern Peru, and midwestern Bolivia. Nicotiana tomentosa is a semi-arbor shrub that grows in sunny or semi-shaded areas such as riverbanks, valleys, and wastelands, reaching a height of several meters. The stem is thick and sticky, and the leaf has an extremely large egg shape to elliptic shape. The back side of the leaf is covered with fine fluff and is sticky. The number of days until blooming is long. The corolla is about 3 cm long, the corolla tube is pale yellow green, the upper end swells and tilts abruptly into a cup shape, the upper part has a crenation shape, and the fissure has deep parts and shallow parts and is pink, white, or red. The main alkaloids are nornicotine and anabasine. Nicotiana tomentosa is resistant to powdery mildew, PVY, TEV, and nematode disease (Meloidogyne) (The genus Nicotiana illustrated, Japan Tobacco Inc. (edited), 36-39, 1994 March, SEIBUNDO SHINKOSHA Publishing Co., LTD.).

With respect to hybridization between Nicotiana tomentosa and another species, there are reports as follows.
Takenaka et al., Jap. Jour. Genet. 31. 155-161, 1956
   Nicotiana tomentosa × Nicotiana sylvestris
Greenleaf et al., Sterile and Fertile Amphidiploids: Their Possible Relation to the Origin of Nicotiana Tabacum, Genetics, 26, 301-324, 1941
   Nicotiana sylvestris × Nicotiana tomentosa, Nicotiana glutinosa × Nicotiana tomentosa, and Nicotiana tomentosiformis × Nicotiana tomentosa
Smith et al., Alkaloids in certain species and interspecific hybrids of Nicotiana, Journal of Agricultural Research, Vol. 65(7), 347-359, 1942
   Nicotiana glauca × Nicotiana tomentosa
Tezuka et al., Hybrid lethality in the genus Nicotiana, Botany, 191-210, 2012
   Nicotiana glauca × Nicotiana tomentosa, Nicotiana trigonophylla × Nicotiana tomentosa, Nicotiana palmeri × Nicotiana tomentosa, Nicotiana tomentosa × Nicotiana bigelovii, Nicotiana tomentosa × Nicotiana quadrivalvis, and Nicotiana quadrivalvis × Nicotiana tomentosa

The above-described literatures do not describe the characteristics of the hybrids obtained by hybridization, such as the blooming time, the number of flowers, and the insect resistance. In addition, in crossing between Nicotiana tomentosa and another wild species, it is known that a hybrid created withers without growing (cross fatality) in some cases (for example, Nicotiana glutinosa × Nicotiana tomentosa, Nicotiana tomentosa × Nicotiana bigelovii, and Nicotiana tomentosa × Nicotiana quadrivalvis.) (McCray et al., Compatibility of Certain Nicotiana Species, Genetics, 17(6), 621-636, 1932 and East et al., Genetic reactions in Nicotiana, Genetics, 20, 403-413, 1935).

### CITATION LIST

### PATENT LITERATURE

PTL 1: US Patent No. 7,798,153
PTL 2: International Publication No. WO 2019/139176

### NON PATENT LITERATURE

NPL 1: Takenaka, Jap. Jour. Genet. 31. 155-161, 1956
NPL 2: MULKH RAJ AHUJA, Genetica 47, 865-880, 1962
NPL 3: The genus Nicotiana illustrated, Japan Tobacco Inc. (edited), 180-183, 1994 March, SEIBUNDO SHINKOSHA Publishing Co., LTD.
NPL 4: U. Subushini, Euphytica, 23, 289-293, 1974
NPL 5: U. Subushini at al., Cytologia 39, 403-409, 1974
NPL 6: U. Subushini at al., Cytologia 40, 409-413, 1975
NPL 7: BULLETIN OF THE IWATA TOBACCO EXPERIMENT STATION, 17, 1-69, 1985 March
NPL 8: The genus Nicotiana illustrated, Japan Tobacco Inc. (edited), 36-39, 1994 March, SEIBUNDO SHINKOSHA Publishing Co., LTD.)
NPL 9: Takenaka et al., Jap. Jour. Genet. 31. 155-161, 1956
NPL 10: Greenleaf et al., Genetics, 26, 301-324, 1941
NPL 11: Smith et al., Journal of Agricultural Research, Vol. 65(7), 347-359, 1942
NPL 12: Tezuka et al., Botany, 191-210, 2012
NPL 13: McCray et al., Genetics, 17(6), 621-636, 1932
NPL 14: East et al., Genetics, 20, 403-413, 1935

### SUMMARY OF INVENTION

### TECHNICAL PROBLEM

When Nicotiana umbratica is utilized as a raw material for a tobacco product, there are major problems such as (i) the plant body is small and the yielding ability is low and (ii) flower buds are formed at every axillary bud/branch, and therefore topping operation (cutting flower buds) which is carried out for inducing maturation of leaves in ordinary cultivation of tobacco is complicated. Means for solving these problems without impairing the properties of Nicotiana umbratica is unknown from the information obtained from the literatures.

As one solution, it is supposed to utilize an F1 hybrid which is obtained by hybridizing Nicotiana umbratica and another plant of the genus Nicotiana, but a problem such that a hybrid seed is not obtained, or germination does not occur even though a hybrid seed is obtained is brought about depending on the combination in hybridization. Further, much labor is needed for picking out a mate satisfying the condition for obtaining an F1 hybrid in which yield ability and complexity in topping operation are ameliorated without impairing a characteristically strong scent of Nicotiana umbratica.

An object of the present invention is to provide an F1 hybrid plant in which the yield ability and the complexity in topping operation are ameliorated without impairing the characteristically strong scent of Nicotiana umbratica (control variety 1), and a method for making the F1 hybrid plant.

The present inventors have conducted hybridization between Nicotiana umbratica and a plurality of wild species of the genus Nicotiana and investigated the properties of resultant F1 hybrids in detail. As a result, the present inventors have found that an F1 hybrid obtained using a combination of Nicotiana umbratica and Nicotiana kawakamii has excellent characteristics for obtaining a raw material for a tobacco product, the characteristics being such that the yield ability and the complexity in topping operation are ameliorated as compared to Nicotiana umbratica without impairing the characteristically strong scent of Nicotiana umbratica. The present inventors filed International Application No. PCT/JP 2019/001363 on the F1 hybrid using the combination of Nicotiana umbratica and Nicotiana kawakamii, and the application was published on July 18, 2019 (International Publication No. WO 2019/139176). The present application is an application within one year from International Publication No. WO 2019/139176 of International Application No. PCT/JP 2019/001363, in which inventors and applicant are the same as the present application.

The F1 hybrid between Nicotiana umbratica and Nicotiana kawakamii has a large number of axillary buds (a large number of branches) from the base of the leaves after blooming. Thereafter, the apex of each axially bud has flowers, and further, axially buds are generated from the base of the leaves of the grown axillary buds, and flowers bloom at the apexes in the same way. In the F1 hybrid between Nicotiana umbratica and Nicotiana kawakamii, when compared to Nicotiana umbratica, amelioration can be seen such as a ratio (%) of height of a lowermost flower to grass height increases as compared to Nicotiana umbratica, the number of flowers per plant is smaller than that of Nicotiana umbratica, the number of branches decreases as compared to that of Nicotiana umbratica. However, topping operation (cutting flower buds/stems) which is carried out for inducing maturation of harvested leaves in ordinary cultivation of tobacco is still needed and complicated although it has been ameliorated in the F1 hybrid as compared to Nicotiana umbratica.

An object of the present invention is to provide an F1 hybrid plant in which the number of flowers per plant decreases and the complexity in topping operation are ameliorated as compared to control variety 1 or control variety 2 without impairing the characteristic scent of Nicotiana umbratica (control variety 1) or the F1 hybrid (control variety 2) between Nicotiana umbratica and Nicotiana kawakamii, and a method for making the F1 hybrid plant.

### SOLUTION TO PROBLEM

The present invention includes, but not limited to, the following embodiments.
[Embodiment 1] An F1 hybrid between Nicotiana umbratica and Nicotiana tomentosa, or a portion thereof.
[Embodiment 2] The F1 hybrid or a portion thereof according to embodiment 1, wherein the portion is selected from the group consisting of a leaf, a stem, a root, a seed, a flower, pollen, an anther, an ovule, a pedicel, a meristematic tissue, a seed leaf, an embryonic axis, a pericycle, an embryo, an endosperm, an explant tip, a callus, a tissue-cultured product, a bud, a cell, and a protoplast.
[Embodiment 3] The F1 hybrid or a portion thereof according to embodiment 1 or 2, wherein a seed parent is Nicotiana umbratica, and a pollen parent is Nicotiana tomentosa.
[Embodiment 4] The F1 hybrid or a portion thereof according to embodiment 1 or 2, wherein a pollen parent is Nicotiana umbratica, and a seed parent is Nicotiana tomentosa.
[Embodiment 5] The F1 hybrid or a portion thereof according to any one of embodiments 1 to 4, wherein a number of flowers per plant is smaller than that of an F1 hybrid between Nicotiana umbratica and Nicotiana kawakamii.
[Embodiment 6] The F1 hybrid or a portion thereof according to any one of embodiments 1 to 5, wherein insect resistance against a defoliator pest is improved as compared to an F1 hybrid between Nicotiana umbratica and Nicotiana kawakamii.
[Embodiment 7] The F1 hybrid or a portion thereof according to embodiment 6, wherein the defoliator pest is selected from the group consisting of oriental tobacco budworm (Helicoverpa assulta), tobacco budworm (Heliothis virescens), cotton bollworm (Helicoverpa armigera), tobacco hornworm (Manduca sexta), Spodoptera litura, and Spodoptera exigua.
[Embodiment 8] The F1 hybrid or a portion thereof according to any one of embodiments 1 to 7, wherein insect resistance against a stored-tobacco pest is improved as compared to an F1 hybrid between Nicotiana umbratica and Nicotiana kawakamii.
[Embodiment 9] A method for making an F1 hybrid, comprising hybridizing Nicotiana umbratica and Nicotiana tomentosa.
[Embodiment 10] The method according to embodiment 9, wherein a seed parent is Nicotiana umbratica, and a pollen parent is Nicotiana tomentosa.
[Embodiment 11] The method according to embodiment 10, wherein a pollen parent is Nicotiana umbratica, and a seed parent is Nicotiana tomentosa.
[Embodiment 12] The method according to any one of embodiments 9 to 11, further comprising a step of choosing the F1 hybrid having a number of flowers per plant smaller than the number of flowers of an F1 hybrid between Nicotiana umbratica and Nicotiana kawakamii after hybridizing Nicotiana umbratica and Nicotiana tomentosa.
[Embodiment 13] A composition comprising the F1 hybrid or a portion thereof according to any one of embodiments 1 to 8, or a composition comprising an extract of the F1 hybrid or a portion thereof according to any one of embodiments 1 to 8.
[Embodiment 14] The composition according to embodiment 13, being in a cut filler, powder, sheet, or solution form.
[Embodiment 15] A product comprising the F1 hybrid or a portion thereof according to any one of embodiments 1 to 8, or a product comprising an extract of the F1 hybrid or a portion thereof according to any one of embodiments 1 to 8.
[Embodiment 16] A product comprising the composition according to embodiment 13 or 14.
[Embodiment 17] The product according to embodiment 15 or 16, selected from the group consisting of a tobacco product, a perfume, an agricultural chemical, and a pharmaceutical product.
[Embodiment 18] The F1 hybrid or a portion thereof according to any one of embodiments 1 to 8, or an extract of the F1 hybrid or a portion thereof according to any one of embodiments 1 to 8 for use for producing a composition or a product.
[Embodiment 19] Use of the F1 hybrid or a portion thereof according to any one of embodiments 1 to 8, or use of an extract of the F1 hybrid or a portion thereof according to any one of embodiments 1 to 8 for producing a composition or a product.
[Embodiment 20] The F1 hybrid or a portion thereof according to any one of embodiments 1 to 4, wherein a number of flowers per plant is smaller than that of Nicotiana umbratica.
[Embodiment 21] The F1 hybrid or a portion thereof according to any one of embodiments 1 to 4 and 20, wherein a number of branches decreases as compared to the number of branches of Nicotiana umbratica.
[Embodiment 22] The F1 hybrid or a portion thereof according to any one of embodiments 1 to 4 and 20 to 21, wherein a yield of an above-ground part increases as compared to Nicotiana umbratica.
[Embodiment 23] The F1 hybrid or a portion thereof according to any one of embodiments 1 to 4 and 20 to 22, wherein insect resistance against a defoliator pest is improved as compared to Nicotiana tomentosa.
[Embodiment 24] The F1 hybrid or a portion thereof according to any one of embodiments 1 to 4 and 20 to 23, wherein insect resistance against a defoliator pest is improved as compared to both of Nicotiana tomentosa and Nicotiana umbratica.
[Embodiment 25] The F1 hybrid or a portion thereof according to embodiment 23 or 24, wherein the defoliator pest is selected from the group consisting of oriental tobacco budworm (Helicoverpa assulta), tobacco budworm (Heliothis virescens), cotton bollworm (Helicoverpa armigera), tobacco hornworm (Manduca sexta), Spodoptera litura, and Spodoptera exigua.
[Embodiment 26] The F1 hybrid or a portion thereof according to any one of embodiments 1 to 4 and 20 to 25, wherein insect resistance against a stored-tobacco pest is improved as compared to Nicotiana tomentosa.
[Embodiment 27] The F1 hybrid or a portion thereof according to any one of embodiments 1 to 4 and 20 to 26, wherein insect resistance against a stored-tobacco pest is improved as compared to both of Nicotiana tomentosa and Nicotiana umbratica.
[Embodiment 28] The method according to any one of embodiments 9 to 11, further comprising a step of choosing the F1 hybrid having a number of flowers per plant smaller than the number of flowers of Nicotiana umbratica after hybridizing Nicotiana umbratica and Nicotiana tomentosa.
[Embodiment 29] A composition comprising the F1 hybrid or a portion thereof according to any one of embodiments 1 to 4 and 20 to 27, or a composition comprising an extract of the F1 hybrid or a portion thereof according to any one of embodiments 1 to 4 and 20 to 27.
[Embodiment 30] The composition according to embodiment 29, being in a cut filler, powder, sheet, or solution form.
[Embodiment 31] A product comprising the F1 hybrid or a portion thereof according to any one of embodiments 1 to 4 and 20 to 27, or a product comprising an extract of the F1 hybrid or a portion thereof according to any one of embodiments 1 to 4 and 20 to 27.
[Embodiment 32] A product comprising the composition according to embodiment 29 or embodiment 30.
[Embodiment 33] The product according to embodiment 31 or 32, selected from the group consisting of a tobacco product, a perfume, an agricultural chemical, and a pharmaceutical product.

### ADVANTAGEOUS EFFECTS OF INVENTION

An F1 hybrid obtained by hybridizing Nicotiana umbratica and Nicotiana tomentosa is also an F1 hybrid in which the number of flowers per plant decreases and the complexity in topping operation is ameliorated as compared to control variety 1 and control variety 2 without impairing the characteristic scent of Nicotiana umbratica (control variety 1) or the F1 hybrid (control variety 2) between Nicotiana umbratica and Nicotiana kawakamii.

The F1 hybrid obtained by hybridizing Nicotiana umbratica and Nicotiana tomentosa is an F1 hybrid in which the yield ability and the complexity in topping operation are ameliorated without impairing the characteristically strong scent of Nicotiana umbratica (control variety 1).

Special technique/know-how are unnecessary for cultivation management of the F1 hybrid, and the F1 hybrid can easily be cultivated until harvest by almost the same management as in general Nicotiana tabacum.

The problems of conventional techniques can remarkably be ameliorated by making an F1 hybrid between wild species using Nicotiana umbratica and Nicotiana tomentosa and utilizing the F1 hybrid that improves the yield and the easiness of removing the flower part without impairing the characteristic scent of Nicotiana umbratica. The F1 hybrid further has an effect of enabling remarkable suppression of the extent of being eaten by insects as compared to Nicotiana umbratica (control variety 1) or the F1 hybrid (control variety 2) between Nicotiana umbratica and Nicotiana kawakamii.

### BRIEF DESCRIPTION OF DRAWINGS

Fig. 1 is a photograph showing appearances of the whole plant bodies of an F1 hybrid (control variety 2) between Nicotiana umbratica and Nicotiana kawakamii and an F1 hybrid tobacco plant (F1 hybrid) between Nicotiana umbratica and Nicotiana tomentosa 100 days after seeding.
Fig. 2 is a bar graph showing a weight (g) (average value) per plant of control variety 2 and an F1 hybrid.
Fig. 3 is a bar graph showing a number of terminal buds (/plant) of control variety 2 and an F1 hybrid.
Fig. 4 is a bar graph showing a number of flowers of control variety 2 and an F1 hybrid.
Fig. 5 includes a photograph (left) showing an arrangement of leaves of each plant of an F1 hybrid and control variety 2, the photograph (left) showing a state at the start of an experiment, and a photograph (right) showing a state 40 hours after larvae of oriental tobacco budworm (Helicoverpa assulta) are placed in an investigation on insect resistance against larvae of oriental tobacco budworm (Helicoverpa assulta) in Example 2.
Fig. 6 is a graph showing a change over time of a ratio (%) of an area of leaves after scattering insects to an area of leaves before scattering insects in Example 2.
Fig. 7 is a bar graph showing a relative value of each component in an F1 hybrid to that in control variety 2 (assuming the amount of each component in control variety 2 to be 1) in Example 3.

3-hexenal,
IVA: isovaleric acid (IVA),
3MVA: 3-methylvaleric acid,
4MHA: 4-methylhexanoic acid,
terpene compound A: (RI (Retention index) 1617) (1-isopropyl-5-(hydroxymethyl)-8-methyltricyclo[4.4.0.02,8]dec-4-ene), and
labadadiene-8-ol

### DESCRIPTION OF EMBODIMENTS

The present invention includes, but not limited to, the following embodiments.

### 1. F1 hybrid

The present invention relates to an F1 hybrid between Nicotiana umbratica and Nicotiana tomentosa, or a portion thereof.

"Nicotiana umbratica" is a species of the genus Nicotiana, spreading in northwestern Western Australia. The chromosome number (2n) is 46. Nicotiana umbratica has a grass height of about 50 to about 60 cm, grows thick while a large number of thin stems branch, and has a large number of small leaves of about 5 to about 15 cm, although not limited thereto. Nicotiana umbratica has a lot of racemose flowers at the tips of branches. Nicotiana umbratica as the whole plant body has a large secretion quantity from trichomes to be sticky, and a strong scent floats from the whole, although not limited thereto. The main alkaloid component is nicotine.

"Nicotiana tomentosa" is a species of the genus Nicotiana, spreading in central to southern Peru, and midwestern Bolivia. The chromosome number (2n) is 24. The grass height of Nicotiana tomentosa becomes 200 cm or more in average, and the leaf is extremely large, although not limited thereto. The main alkaloid components are nornicotine anabasine.

"Nicotiana kawakamii" is a species discovered by Kawakami Yoshiyasu et al. in Bolivia in 1968. The chromosome number (2n) is 24. The grass height of Nicotiana kawakamii becomes 200 cm or more in average, and the leaf is large, although not limited thereto. The main alkaloid component is nornicotine.

The "F1 hybrid" is progeny born from plants whose species or varieties are different. The "F1 hybrid" in the present specification and the claims of the present application means an F1 hybrid as the first filial generation (F1). F1 is the first generation progeny generated as a result of hybridization between parents having certain different alleles as being homozygous in a living thing. F1 has genes of the parents as being heterozygous, and the genotype is uniform. The chromosome number (2n) is 35.

The F1 hybrid in the present specification means the "F1 hybrid between Nicotiana umbratica and Nicotiana tomentosa" unless otherwise noticed.

The portion of the F1 hybrid in the present invention includes not only a portion of an adult of a plant but also all the embodiments from a seed to an adult of a plant.

The "portion" is selected from the group consisting of a leaf (including a leaf blade and a leafstalk), a stem, a root, a seed, a flower, pollen, an anther, an ovule, a pedicel, a meristematic tissue, a seed leaf, an embryonic axis, a pericycle, an embryo, an endosperm, an explant tip, a callus, a tissue-cultured product, a bud, a cell, and a protoplast, although not limited thereto. The portion is preferably a leaf or a stem.

The "portion" is a nonproliferative portion of the F1 hybrid, although not limited thereto. The "nonproliferative portion" includes a leaf (including a leaf blade and a leafstalk), a stem, a root, a flower, a pedicel, a pericycle, an explant tip, a callus, and a tissue-cultured product which are isolated from a plant body, although not limited thereto.

The "leafstalk" is a small shaft that connects a leaf blade and a stem in a plant. The "pedicel" is a short shaft that joins a single-petaled flower and a stem which leads to inflorescence in a seed plant. The "pericycle" is a tissue of a plant and is a layer of cells, the layer surrounding a xylem where the center of a root exists and a phloem. When the pericycle divides, a lateral root is thereby formed. The "explant tip" is a tissue slice of a plant, the tissue slice obtained by culturing under an artificial condition a portion of an embryo cut out from the plant. The "callus" is a lump of undifferentiated plant cells which are being cultured on a solid medium or the like. The "tissue-cultured product" is a cultured product of a plant tissue obtained by culturing an arbitrary tissue of a plant under an artificial condition.

In the F1 hybrid, any of Nicotiana umbratica and Nicotiana tomentosa may be a seed parent or may be a pollen parent.

In one embodiment, the seed parent is Nicotiana umbratica, and the pollen parent is Nicotiana tomentosa. In another embodiment, the pollen parent is Nicotiana umbratica, and the seed parent is Nicotiana tomentosa. More preferably, the seed parent is Nicotiana umbratica, and the pollen parent is Nicotiana tomentosa.

### 2. Characteristics of F1 hybrid

### (1) Number of Flowers

To efficiently collect the above-ground part including leaves and stems each useful in order to be used for producing a tobacco product from a tobacco plant, it is necessary to remove the flowers from the tobacco plant. Removal of flowers is easier in the F1 hybrid than in Nicotiana umbratica.

In one embodiment, the number of flowers per plant at the harvest time for leaf tobacco is smaller in the F1 hybrid than that of the F1 hybrid between Nicotiana umbratica and Nicotiana kawakamii. This is because the number of terminal buds in the F1 hybrid is almost equal to that in the F1 hybrid between Nicotiana umbratica and Nicotiana kawakamii, but after the first blooming, the F1 hybrid blooms much later than the F1 hybrid between Nicotiana umbratica and Nicotiana kawakamii (the F1 hybrid blooms at or after the time for harvesting leaves), although not limited thereto. In one embodiment, the number of flowers per plant is smaller in the F1 hybrid than that of Nicotiana umbratica.

In Example 1, which will be described later, the "number of flowers" per plant about 100 days after transplantation decreased in the F1 hybrid to about one thirtieth of that in the F1 hybrid (control variety 2) between Nicotiana umbratica and Nicotiana kawakamii. The number of flowers per plant in the F1 hybrid is preferably one half or less, one fifth or less, one tenth or less, one twentieth or less, or one thirtieth or less of that in control 2. The number of flowers per plant in the F1 hybrid is preferably one tenth or less, one twentieth or less, one thirtieth or less, one fiftieth or less, one hundredth, or one hundred and fiftieth or less of that in Nicotiana umbratica (control variety 1).

In the F1 hybrid, the number of flowers is small, and therefore removal of the flowers and topping operation (cutting flower buds and stems) are easy.

### (2) Number of Branches

In one embodiment, the number of branches in the F1 hybrid is almost equal to that in the F1 hybrid between Nicotiana umbratica and Nicotiana kawakamii. In one embodiment, the number of branches decreases in the F1 hybrid as compared to Nicotiana umbratica.

Harvest of leaves for the F1 hybrid is easier than for the F1 hybrid between Nicotiana umbratica and Nicotiana kawakamii and for Nicotiana umbratica.

### (3) Harvest of Above-ground Part

The above-ground part including leaves and stems each useful in order to be used for producing a tobacco product can preferably be harvested more in the F1 hybrid than in Nicotiana umbratica.

In one embodiment, the yield of the above-ground part increases in the F1 hybrid as compared to Nicotiana umbratica.

The yield of the above-ground part per plant is preferably 1.2 times or more, 1.5 times or more, 1.8 times or more, 2 times or more, or 2.1 times or more as compared to Nicotiana umbratica.

In one embodiment, the yield of dried leaves increases in the F1 hybrid as compared to Nicotiana umbratica. The "dried leaves" are leaves obtained by subjecting the leaves of a tobacco plant to a drying treatment, such as freeze drying, drying by heating, drying in the sun, or air drying, thereby drying the leaves of the tobacco plant.

### (4) Insect Resistance Against Pest

The F1 hybrid preferably has strong resistance against a pest.

It is generally known that Nicotiana tomentosa and Nicotiana kawakamii which are wild species have weak insect resistance against a lot of pests, and Nicotiana umbratica has strong insect resistance against a pest.

In one embodiment, the insect resistance against a defoliator pest is improved in the F1 hybrid as compared to the F1 hybrid between Nicotiana umbratica and Nicotiana kawakamii.

In one embodiment, the insect resistance against a defoliator pest is improved in the F1 hybrid as compared to Nicotiana tabacum. In one embodiment, the insect resistance against a defoliator pest is improved in the F1 hybrid as compared to Nicotiana tomentosa. More preferably, the insect resistance against a defoliator pest is improved in the F1 hybrid as compared to both of Nicotiana tomentosa and Nicotiana umbratica.

In the present specification and the appended Claims, the "pest" is used as a general term of an arbitrary insect that is harmful to a tobacco plant. Examples of the defoliator pest include oriental tobacco budworm (Helicoverpa assulta), tobacco budworm (Heliothis virescens), cotton bollworm (Helicoverpa armigera), tobacco hornworm (Manduca sexta), Spodoptera litura, and Spodoptera exigua.

"Oriental tobacco budworm (Helicoverpa assulta)" the larva (green caterpillar) of which in particular eats various farm products is particularly known as a pest against solanaceous plants, such as tobacco and a sweet pepper, fruits, flowers, and the like. The "green caterpillar" is a green larva the body of which is not covered with long hair among the larvae of Lepidoptera (butterflies and moths).

In one embodiment, the insect resistance against a stored-tobacco pest is improved in the F1 hybrid as compared to the F1 hybrid between Nicotiana umbratica and Nicotiana kawakamii.

In one embodiment, the insect resistance against a stored-tobacco pest is improved in the F1 hybrid as compared to Nicotiana tabacum. In one embodiment, the insect resistance against a stored-tobacco pest is improved in the F1 hybrid as compared to Nicotiana tomentosa. In one embodiment, the insect resistance against a stored-tobacco pest is improved in the F1 hybrid as compared to both of Nicotiana tomentosa and Nicotiana umbratica.

Examples of the stored-tobacco pest include pests that eat dried leaves of tobacco, such as Lasioderma serricorne and Ephestia elutella.

### (5) Components Contained in Tobacco

Preferably, all or part of components that contribute to the characteristic flavor (preferred flavor as tobacco) does not decrease in the F1 hybrid as compared to the F1 hybrid between Nicotiana umbratica and Nicotiana kawakamii. Preferably, all or part of components that contribute to the characteristic flavor (preferred flavor as tobacco) does not decrease in the F1 hybrid as compared to Nicotiana umbratica.

Examples of the components that contribute to the preferred scent as tobacco include 1-isopropyl-5-(hydroxymethyl)-8-methyltricyclo[4.4.0.02,8]dec-4-ene and 4-methylhexanoic acid.

Accordingly, the F1 hybrid can be used for a product, such as a tobacco product or a perfume, while retaining the preferred flavor of Nicotiana umbratica.

### 3. Method for Making F1 hybrid

The present invention provides a method for making an F1 hybrid in one embodiment. The method of the present invention includes hybridizing Nicotiana umbratica and Nicotiana tomentosa. A known method for hybridizing tobacco plants can be used for the hybridization.

The definition of the "F1 hybrid" is as described in "1. F1 hybrid".

In the method for making an F1 hybrid of the present invention, any of Nicotiana umbratica and Nicotiana tomentosa may be a seed parent or may be a pollen parent.

In one embodiment, the seed parent is Nicotiana umbratica, and the pollen parent is Nicotiana tomentosa. In another embodiment, the pollen parent is Nicotiana umbratica, and the seed parent is Nicotiana tomentosa. More preferably, the seed parent is Nicotiana umbratica, and the pollen parent is Nicotiana tomentosa.

In one embodiment of the method for making an F1 hybrid of the present invention, a step of choosing the F1 hybrid having a more preferred characteristic may further be included after hybridizing Nicotiana umbratica and Nicotiana tomentosa. The step of choosing the F1 hybrid includes, but not limited to, a step of choosing F1 hybrid having a smaller number of flowers per plant than the F1 hybrid between Nicotiana umbratica and Nicotiana kawakamii.

### 4. Composition

The present invention provides a composition containing the F1 hybrid or a portion thereof, or a composition containing an extract of the F1 hybrid or a portion thereof in one embodiment.

The definitions of the "F1 hybrid" and "portion" are as described in "1. F1 hybrid".

With respect to the composition, all or a portion of a tobacco plant may be used as it is, or all or a portion of a tobacco plant cut, pulverized, or ground into a slender piece-like, slurry-like, or fine particle-like product may be used. With respect to the composition, all or a portion of a tobacco plant harvested from farmland or the like may be used as it is, all or a portion of a tobacco plant obtained by being left to stand indoors or outdoors for a predetermined period of time to dissipate part of moisture may be used, or all or a portion of a tobacco plant obtained by almost dissipating moisture with a drier or the like may be used.

In one embodiment, the composition contains a nonproliferative portion of the F1 hybrid.

The "extract" is obtained by subjecting a tobacco plant-derived material (including a "portion", preferably a "nonproliferative portion"), such as leaves or stems, to extraction for the purpose of ameliorating the flavor of a tobacco product or for the purpose of lowering the contents of particular components in a tobacco product. As an extraction method, a known method for extracting refined oil, a particular component, or the like from a plant can be used.

The composition is in a cut filler, powder, sheet, or solution form, although not limited thereto.

In one embodiment, the composition preferably contains about the same amount of a component that contributes to the characteristic flavor (preferred flavor as tobacco) as in a composition (control 1) containing the F1 hybrid between Nicotiana umbratica and Nicotiana kawakamii or a portion thereof, or a composition (control 1) containing an extract of the F1 hybrid between Nicotiana umbratica and Nicotiana kawakamii or a portion thereof. In one embodiment, the composition preferably contains about the same amount of a component that contributes to the characteristic flavor (preferred flavor as tobacco) as in a composition (control 2) containing Nicotiana umbratica or a portion thereof, or a composition (control 2) containing an extract of Nicotiana umbratica or a portion thereof. Being "about the same" means that the composition of the present invention, when compared to control 1 or control 2 prepared in the same manner using substantially the same amount of the materials, contains about 80% to about 120%, more preferably about 90% to about 110%, of the component that contributes to the preferred scent as tobacco, although not limited thereto.

Examples of the component that contributes to the preferred scent as tobacco include 1-isopropyl-5-(hydroxymethyl)-8-methyltricyclo[4.4.0.02,8]dec-4-ene and 4-methylhexanoic acid.

The composition of the present invention contains 1-isopropyl-5-(hydroxymethyl)-8-methyltricyclo[4.4.0.02,8]dec-4-ene and/or 4-methylhexanoic acid in one embodiment.

The present invention includes a method for producing a composition containing an extract of the F1 hybrid or a portion thereof in one embodiment. The method for producing a composition includes, but not limited to, extracting a tobacco plant-derived material from the F1 hybrid between Nicotiana umbratica and Nicotiana tomentosa.

The present invention includes a tobacco material containing the F1 hybrid or a portion thereof, or a tobacco material containing an extract of the F1 hybrid or a portion thereof in one embodiment. The "tobacco material" is synonymous with the "composition" that is used for a tobacco product.

### 5. Product

The above described F1 hybrid or a portion thereof, or an extract of the F1 hybrid or a portion thereof, and the composition containing the F1 hybrid or a portion thereof or the composition containing an extract of the F1 hybrid or a portion thereof have characteristic flavor derived from Nicotiana umbratica or the F1 hybrid between Nicotiana umbratica and Nicotiana tomentosa. Accordingly, in one embodiment, the characteristic flavor derived from Nicotiana umbratica or the F1 hybrid between Nicotiana umbratica and Nicotiana tomentosa can be imparted to a product by using any of these as a raw material for a tobacco product or the like.

The present invention provides a product including the above-described F1 hybrid or a portion thereof, or a product including an extract of the above-described F1 hybrid or a portion thereof in one embodiment.

The present invention provides a product including a composition containing the above-described F1 hybrid or a portion thereof, or a product including a composition containing an extract of the above-described F1 hybrid or a portion thereof in one embodiment. The product includes, but not limited to, a tobacco product, a perfume, an agricultural chemical, and a pharmaceutical product.

In one embodiment, the product includes a nonproliferative portion of the F1 hybrid.

The type of the "tobacco product" is not particularly limited. In addition to a cigarette, a cigar, a pipe tobacco, snuff, a chewing tobacco, a shredded tobacco (including a finely shredded tobacco), a water pipe, and the like are included. Further, a heating type flavor inhaler using, as an aerosol source, aerosol which is generated by heating tobacco, a non-heating type flavor inhaler for inhaling the flavor of tobacco without heating the tobacco, and the like are also included.

The types of the perfume and the agricultural chemical are not limited, too. Examples of the agricultural chemical include agricultural chemicals intended for control of a germ, a nematode, a tick, an insect, a mouse, and other animals and plants, or a virus that harms "farm products", and promotion or inhibition of physiological functions, or inhibition of germination of farm products and the like. The perfume, agricultural chemical, and pharmaceutical product can be used singly or together with another active ingredient.

In one embodiment, the product preferably includes about the same amount of a component that contributes to the characteristic flavor (preferred flavor as tobacco) as in a product (control 1) including the F1 hybrid between Nicotiana umbratica and Nicotiana kawakamii or a portion thereof, or a product (control 1) including an extract of the F 1 hybrid between Nicotiana umbratica and Nicotiana kawakamii or a portion thereof. In one embodiment, the product preferably includes about the same amount of a component that contributes to the characteristic flavor (preferred flavor as tobacco) as in a product (control 2) including Nicotiana umbratica or a portion thereof, or a product (control 2) including an extract of Nicotiana umbratica or a portion thereof. Being "about the same" means that the product of the present invention, when compared to control 1 or control 2 prepared in the same manner using substantially the same amount of the materials, includes about 80% to about 120%, more preferably about 90% to about 110%, of the component that contributes to the preferred scent as tobacco, although not limited thereto.

Examples of the component that contributes to the preferred scent as tobacco include 1-isopropyl-5-(hydroxymethyl)-8-methyltricyclo[4.4.0.02,8]dec-4-ene and 4-methylhexanoic acid.

The product of the present invention includes 1-isopropyl-5-(hydroxymethyl)-8-methyltricyclo[4.4.0.02,8]dec-4-ene and/or 4-methylhexanoic acid in one embodiment.

The present invention provides use of the F1 hybrid or a portion thereof, or an extract of the F1 hybrid or a portion thereof for producing a composition or a product in one embodiment.

The present invention includes a method for producing a tobacco product in one embodiment. The method for producing a tobacco product includes, but not limited to, extracting a tobacco plant-derived material from the F1 hybrid between Nicotiana umbratica and Nicotiana tomentosa. Here, the tobacco product is selected from the group consisting of a cigarette, a cigar, a pipe tobacco, snuff, a chewing tobacco, a shredded tobacco (including a finely shredded tobacco), a water pipe, a heating type flavor inhaler, and a non-heating type flavor inhaler.

### EXAMPLES

Hereinafter, the present invention will be described in detail based on Examples, but the present invention is not limited to these Examples. A person skilled in the art can easily modify/change the present invention based on the description of the present specification, and such modifications and changes are included in the technical scope of the present invention.

### Example 1 Making F1 hybrid between Nicotiana umbratica and Nicotiana tomentosa, and Properties of Same

In the present Example, an F1 hybrid between Nicotiana umbratica and Nicotiana tomentosa was made.

Hybridization was performed by a known method using Nicotiana umbratica (obtained from Dr. E. L. Moore of United States of America in 1967) as a seed parent and Nicotiana tomentosa (obtained from Dr. D. R. Cameron of United States of America in 1963) as a pollen parent. The objective F1 hybrid was also obtained when the seed parent and the pollen parent were replaced with each other.

Hybridization was performed by a known method using Nicotiana umbratica as a seed parent and Nicotiana kawakamii (obtained from the Central Andes academic investigation team of Kyoto University in 1976) as a pollen parent. The F1 hybrid between these was also obtained when the seed parent and the pollen parent were replaced with each other.

Hereinafter, in the present Example, detailed description will be made taking the case where Nicotiana umbratica was used as a seed parent, and Nicotiana tomentosa was used as a pollen parent as an example. The case where Nicotiana umbratica was used as a seed parent, and Nicotiana kawakamii was used as a pollen parent was adopted as an example of control variety 2.

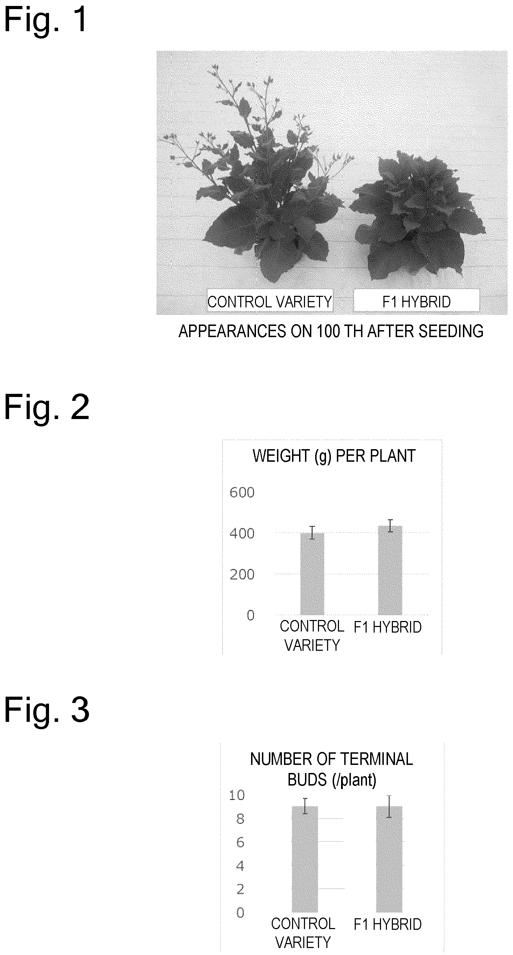

The F1 hybrid was cultivated in a greenhouse of JT Leaf Tobacco Research Center (Oyama-shi, Tochigi). Super Mix (SAKATA SEED CORPORATION) was used as the soil for seeding, and the F1 hybrid was transplanted into Super Culture Soil (SunAgro CO., Ltd.), which is another type of soil for raising seedlings, about 20 days after seeding. A seedling suitable for transplantation was obtained about 40 days after seeding. Vermiculite was packed into a plastic pot having a volume of about 3L up to 70% of the volume to transplant the seedling. Before the transplantation, the vermiculite was impregnated with a sufficient amount of water, and after transplantation, culture solutions (OAT House No. 1 and OAT House No. 2 (OAT Agrio Co., Ltd.), about 200 to about 500 ppm) containing fertilizers such as nitrogen were applied according to everyday weather. As Comparative Examples, the F1 hybrid (control variety 2) between Nicotiana umbratica and Nicotiana kawakamii was also cultivated in the same manner. It is to be noted that the "control variety" described in Figs. 1-7 below represents the F1 hybrid (control variety 2) between Nicotiana umbratica and Nicotiana kawakamii.

The first flower immediately before blooming was cut about 30 days after transplantation, and cultivation was continued for further 30 days (until 100 days after seeding), so that both of the varieties reached the proper time for harvesting tobacco leaves. A photograph of the appearances on the 100th day after seeding is shown in Fig. 1.

Twenty-six plants were cultivated for each of the F1 hybrid and control variety 2 to evaluate each item of the yield (weight per plant), the number of terminal buds, and the number of flowers. The results are shown in Fig. 2 to Fig. 4.

### (1) Yield (Wet Weight of Above-ground Part)

The weight (not dry weight but wet weight) per plant on the 100th day after seeding was as shown in Fig. 2. The "weight per plant" means the weight (wet weight) of an above-ground part before drying, the above-ground part cut and separated from an individual at a position about 3 cm above the ground 100 days after transplantation. It is to be noted that the flower part in this case is removed with some stem parts. The weights per plant of control variety 2 and the F1 hybrid were almost the same. It is to be noted that PTL 2 describes that the yield (weight per plant) of control variety 2 increases as compared to Nicotiana umbratica. The yield of the above-ground part per plant of control variety 2 was about 2 times that of Nicotiana umbratica in Examples of PTL 2.

Accordingly, it was shown that the yield of the F1 hybrid between Nicotiana umbratica and Nicotiana tomentosa was about the same as that of the F1 hybrid between Nicotiana umbratica and Nicotiana kawakamii and increased as compared to Nicotiana umbratica.

### (2) Number of Terminal Buds and Number of Flowers

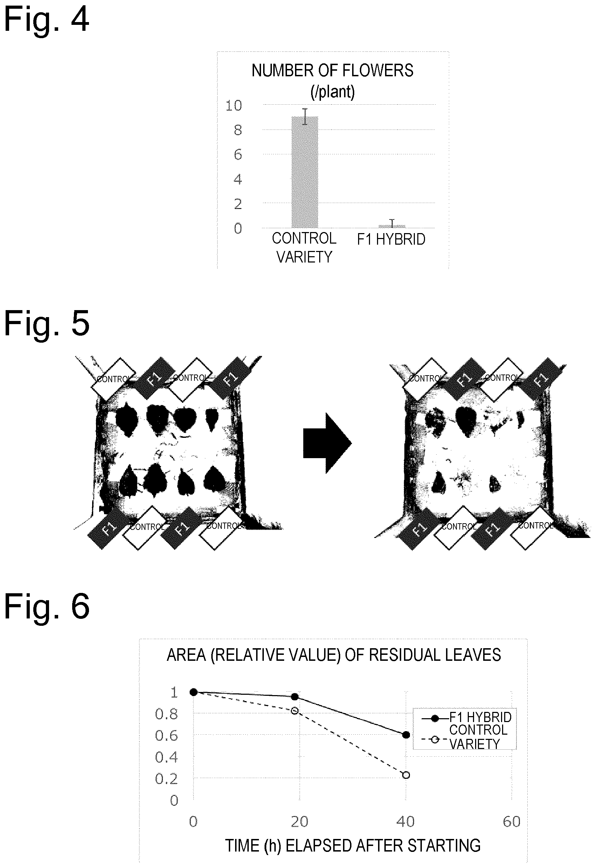

The number of terminal buds and the number of flowers of the F1 hybrid and control variety 2 on the 100th day after seeding are shown in Fig. 3 and Fig. 4.

The number of terminal buds of the F1 hybrid was equal to that of control variety 2. However, many of the axillary buds of control variety 2 grew and had flowers at the tips thereof, but in contrast, in the F1 hybrid, the blooming after the first blooming was much delayed and the number of flowers which bloomed remarkably decreased to about one thirtieth of that of control variety 2. From those described above, it was made clear that the labor for removing the flowers during cultivation can be reduced in the F1 hybrid. It is to be noted that the number of flowers of control variety 2 decreased to about one fifth of that of Nicotiana umbratica in Examples of PTL 2.

It is to be noted that control variety 2 had flowers which had bloomed in all of 8 to 10 terminal buds/plant at all the lower parts (proportion of the terminal buds having flowers which had bloomed among all the terminal buds = 100%). The F1 hybrid had flowers which had bloomed of 0 to 1 terminal buds/plant among 7 to 10 terminal buds/plant (proportion of the terminal buds having flowers which had bloomed among all the terminal buds = about 3%).

Accordingly, it was shown that the number of flowers per plant of the F1 hybrid between Nicotiana umbratica and Nicotiana tomentosa remarkably decreases as compared to control variety 2, and further decreases when compared to Nicotiana umbratica.

From those described above, it was made clear that the labor for topping operation and removal of flowers during cultivation in the F1 hybrid between Nicotiana umbratica and Nicotiana tomentosa can be reduced as compared to the F1 hybrid between Nicotiana umbratica and Nicotiana kawakamii, and can further be reduced as compared to Nicotiana umbratica.

### Example 2 Insect Resistance of F1 hybrid between Nicotiana umbratica and Nicotiana tomentosa against Defoliator Pest

In the present Example, the insect resistance of the F1 hybrid between Nicotiana umbratica and Nicotiana tomentosa against feeding damage by oriental tobacco budworm (Helicoverpa assulta) was investigated in such a way that insects were scattered in a mesh cage in which tobacco leaves were arranged, and the areas of the leaves left after a fixed time were compared. As Comparative Example, control variety 2 was used.

Leaves of the F1 hybrid and control variety 2 were placed in a net cage of 40 cm in width × 40 cm in depth × 40 cm in height together with 20 to 30 larvae of oriental tobacco budworm (Helicoverpa assulta), and the area of the leaves left after 19 hours and 40 hours was compared to that before starting the investigation to investigate the extent of feeding damage. The investigation was carried out in a room with a window out of direct sunlight, and the room temperature was set to about 25 °C with an air conditioner. The F1 hybrid and control variety 2 were (alternately) arranged as shown in Fig. 5 in order to eliminate (minimize) the influence of the type, arrangement, and the like in Comparative Example on the variety selectivity of eating.

The area (relative value) of residual leaves compared to that at the time of starting the investigation is shown in Fig. 6. The larvae of oriental tobacco budworm (Helicoverpa assulta) ate more control variety 2 as compared to the F1 hybrid. Only 23% of the leaves of the control variety were left including natural wilt of the leaves 40 hours after starting the investigation, but 60% of the leaves of the F1 hybrid were left.

It is to be noted that in Examples of PTL 2, similar insect resistance tests against a defoliator pest had already been conducted using control variety 2, Nicotiana umbratica (control variety 1), and other Nicotiana species (Nicotiana kawakamii and/or Nicotiana tabacum (variety: Taihei). As a result, it had already been made clear that control variety 2 has significantly stronger resistance against feeding damage by larvae of oriental tobacco budworm (Helicoverpa assulta) than Nicotiana umbratica.

From those described above, the insect resistance against a defoliator pest is improved in the F1 hybrid between Nicotiana umbratica and Nicotiana tomentosa as compared to the F1 hybrid between Nicotiana umbratica and Nicotiana kawakamii, and is further improved as compared to Nicotiana umbratica.

### Example 3 Component Analysis of F1 hybrid between Nicotiana umbratica and Nicotiana tomentosa

In the present Example, component analysis of the F1 hybrid between Nicotiana umbratica and Nicotiana tomentosa was performed using GC/MS. As Comparative Example, control variety 2 was used.

### [Procedure]

- In a 50 mL centrifuge tube, 4 g of leaves just after being cut from a plant body on the 17th day after transplantation (the 59th day after seeding) are placed.

- Hexane in a volume of 20 mL is added to perform extraction by shaking at 200 rpm for 60 minutes.
- Contents in the centrifuge tube after shaking are filtrated with a filter paper, an appropriate amount of sodium sulfate is added to a filtrate, and a resultant mixture is left to stand for 30 minutes to perform dehydration.
- The liquid after dehydration is filtrated again, and a resultant liquid is then sealed in a vial and used as an analysis sample to perform GC (gas chromatography).
- The total ion chromatogram obtained is analyzed to make relative comparison of the amounts of 3-hexenal, isovaleric acid (IVA), 3-methylvaleric acid (3MVA), 4-methylhexanoic acid (4MHA), terpene compound A (RI (Retention index) 1617) (1-isopropyl-5-(hydroxymethyl)-8-methyltricyclo[4.4.0.02,8]dec-4-ene), and labdadien-8-ol. The amount of each component in control variety 2 is assumed to be 1.

### [Analysis Condition]

Device: Agilent 5977B series GC/MSD system (7980B GC/5977B MSD)
Column: HP-1MS (model number 19091S-733, length (m) 30 × inner diameter (mm) 0.25 × film thickness (µm) 1.00)
Temperature-increasing program: 40°C for 1 minute, 40 to 200°C at 5°C/min, 200 to 220°C at 1°C/min, 220 to 300°C at 5°C/min, 300°C for 21 minutes
Inlet: splitless, 1 µL, 300°C
Detector: Scan mode (m/z 40 to 400)
Terpene compound A (1-isopropyl-5-(hydroxymethyl)-8-methyltricyclo[4.4.0.02,8]dec-4-ene) is a component that contributes to a characteristic scent (preferred scent as tobacco) in Nicotiana umbratica and control variety 2.

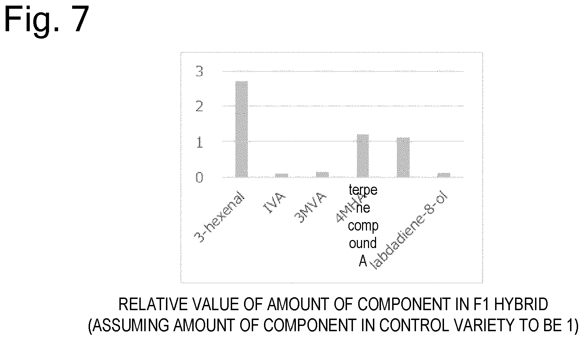

As a result of the component analysis, terpene compound A that is an important aroma component of control variety 2 was also contained in the F1 hybrid in about the same amount as in control variety 2, as shown in Fig. 7. In addition, it had already been ascertained that in Examples of PTL 2, terpene compound A does not decrease in control variety 2 as compared to Nicotiana umbratica (control variety 1).

Accordingly, it was shown that the component that contributes to the characteristic scent (preferred scent as tobacco) does not decrease in the F1 hybrid between Nicotiana umbratica and Nicotiana tomentosa as compared to both of the F1 hybrid between Nicotiana umbratica and Nicotiana kawakamii, and Nicotiana umbratica.

## Claims

1. An F1 hybrid between Nicotiana umbratica and Nicotiana tomentosa, or a portion thereof.

2. The F1 hybrid or a portion thereof according to claim 1, wherein the portion is selected from the group consisting of a leaf, a stem, a root, a seed, a flower, pollen, an anther, an ovule, a pedicel, a meristematic tissue, a seed leaf, an embryonic axis, a pericycle, an embryo, an endosperm, an explant tip, a callus, a tissue-cultured product, a bud, a cell, and a protoplast.

3. The F1 hybrid or a portion thereof according to claim 1 or 2, wherein a seed parent is Nicotiana umbratica, and a pollen parent is Nicotiana tomentosa.

4. The F1 hybrid or a portion thereof according to claim 1 or 2, wherein a pollen parent is Nicotiana umbratica, and a seed parent is Nicotiana tomentosa.

5. The F1 hybrid or a portion thereof according to any one of claims 1 to 4, wherein a number of flowers per plant is smaller than that of an F1 hybrid between Nicotiana umbratica and Nicotiana kawakamii.

6. The F1 hybrid or a portion thereof according to any one of claims 1 to 5, wherein insect resistance against a defoliator pest is improved as compared to an F1 hybrid between Nicotiana umbratica and Nicotiana kawakamii.

7. The F1 hybrid or a portion thereof according to claim 6, wherein the defoliator pest is selected from the group consisting of oriental tobacco budworm (Helicoverpa assulta), tobacco budworm (Heliothis virescens), cotton bollworm (Helicoverpa armigera), tobacco hornworm (Manduca sexta), Spodoptera litura, and Spodoptera exigua.

8. The F1 hybrid or a portion thereof according to any one of claims 1 to 7, wherein insect resistance against a stored-tobacco pest is improved as compared to an F1 hybrid between Nicotiana umbratica and Nicotiana kawakamii.

9. A method for making an F1 hybrid, comprising hybridizing Nicotiana umbratica and Nicotiana tomentosa.

10. The method according to claim 9, wherein a seed parent is Nicotiana umbratica, and a pollen parent is Nicotiana tomentosa.

11. The method according to claim 10, wherein a pollen parent is Nicotiana umbratica, and a seed parent is Nicotiana tomentosa.

12. The method according to any one of claims 9 to 11, further comprising a step of choosing the F1 hybrid having a number of flowers per plant smaller than the number of flowers of an F1 hybrid between Nicotiana umbratica and Nicotiana kawakamii after hybridizing Nicotiana umbratica and Nicotiana tomentosa.

13. A composition comprising the F1 hybrid or a portion thereof according to any one of claims 1 to 8, or a composition comprising an extract of the F1 hybrid or a portion thereof according to any one of claims 1 to 8.

14. The composition according to claim 13, being in a cut filler, powder, sheet, or solution form.

15. A product comprising the F1 hybrid or a portion thereof according to any one of claims 1 to 8, or a product comprising an extract of the F1 hybrid or a portion thereof according to any one of claims 1 to 8.

16. A product comprising the composition according to claim 13 or 14.

17. The product according to claim 15 or 16, selected from the group consisting of a tobacco product, a perfume, an agricultural chemical, and a pharmaceutical product.

18. The F1 hybrid or a portion thereof according to any one of claims 1 to 8, or an extract of the F1 hybrid or a portion thereof according to any one of claims 1 to 8 for use for producing a composition or a product.

19. Use of the F1 hybrid or a portion thereof according to any one of claims 1 to 8, or use of an extract of the F1 hybrid or a portion thereof according to any one of claims 1 to 8 for producing a composition or a product.

20. The F1 hybrid or a portion thereof according to any one of claims 1 to 4, wherein a number of flowers per plant is smaller than that of Nicotiana umbratica.

21. The F1 hybrid or a portion thereof according to any one of claims 1 to 4 and 20, wherein a number of branches decreases as compared to the number of branches of Nicotiana umbratica.

22. The F1 hybrid or a portion thereof according to any one of claims 1 to 4 and 20 to 21, wherein a yield of an above-ground part increases as compared to Nicotiana umbratica.

23. The F1 hybrid or a portion thereof according to any one of claims 1 to 4 and 20 to 22, wherein insect resistance against a defoliator pest is improved as compared to Nicotiana tomentosa.

24. The F1 hybrid or a portion thereof according to any one of claims 1 to 4 and 20 to 23, wherein insect resistance against a defoliator pest is improved as compared to both of Nicotiana tomentosa and Nicotiana umbratica.

25. The F1 hybrid or a portion thereof according to claim 23 or 24, wherein the defoliator pest is selected from the group consisting of oriental tobacco budworm (Helicoverpa assulta), tobacco budworm (Heliothis virescens), cotton bollworm (Helicoverpa armigera), tobacco hornworm (Manduca sexta), Spodoptera litura, and Spodoptera exigua.

26. The F1 hybrid or a portion thereof according to any one of claims 1 to 4 and 20 to 25, wherein insect resistance against a stored-tobacco pest is improved as compared to Nicotiana tomentosa.

27. The F1 hybrid or a portion thereof according to any one of claims 1 to 4 and 20 to 26, wherein insect resistance against a stored-tobacco pest is improved as compared to both of Nicotiana tomentosa and Nicotiana umbratica.

28. The method according to any one of claims 9 to 11, further comprising a step of choosing the F1 hybrid having a number of flowers per plant smaller than the number of flowers of Nicotiana umbratica after hybridizing Nicotiana umbratica and Nicotiana tomentosa.

29. A composition comprising the F1 hybrid or a portion thereof according to any one of claims 1 to 4 and 20 to 27, or a composition comprising an extract of the F1 hybrid or a portion thereof according to any one of claims 1 to 4 and 20 to 27.

30. The composition according to claim 29, being in a cut filler, powder, sheet, or solution form.

31. A product comprising the F1 hybrid or a portion thereof according to any one of claims 1 to 4 and 20 to 27, or a product comprising an extract of the F1 hybrid or a portion thereof according to any one of claims 1 to 4 and 20 to 27.

32. A product comprising the composition according to claim 29 or claim 30.

33. The product according to claim 31 or 32, selected from the group consisting of a tobacco product, a perfume, an agricultural chemical, and a pharmaceutical product.
